# FASCICULE DE BREVET EUROPEEN

(11) **EP 0 724 874 B1**
(45) Date de publication et mention de la délivrance du brevet: **24.04.2002**
(21) Numéro de dépôt: 95101396.0
(22) Date de dépôt: 02.02.1995
(51) Int. Cl.: A61K 7/48

(54) **Mélange d' huiles pour produits cosmétiques**
Aus Ölen bestehende Zusammensetzung für kosmetische Produkte
Mixture of oils for cosmetics

(43) Date de publication de la demande: 07.08.1996
(73) Titulaire: SOCIETE DES PRODUITS NESTLE S.A., 1800 Vevey (CH)
(72) Inventeur: Bertoli, Constantin, CH-1032 Romanel s/Lausanne (CH); Malnoe, Armand, CH-1261 Givrins (CH)
(74) Mandataire: Archambault, Jean

(56) Documents cités:
- WO-A-85/00746
- GB-A- 2 176 713
- US-A- 5 002 760
- DATABASE WPI Week 9033 Derwent Publications Ltd., London, GB; AN 90-249094 & HU-A-51 890 (SZEPESSY G) , 28 Juin 1990
- DR. R. A. ECKSTEIN 'Biokosmetic' 1990 , VERLAG WENNG DRUCK GMBH , DEUTSCHLAND * page 522 * * page 524 - page 525 *
- SOAP, PERFUMERY AND COSMETICS, vol. 64, no. 11, Novembre 1991 LONDON, GB, pages 49-52, 'Waxing Lyrical on Oil'
- PATENT ABSTRACTS OF JAPAN vol. 10 no. 49 (C-330) ,26 Février 1986 & JP-A-60 193911 (SHISEIDO KK) 2 Octobre 1985,
- C. DUVAL, R. DUVAL: "Dictionnaire de la Chimie et de ses Applications,3e édition", 1978, TECHNIQUE ET DOCUMENTATION, PARIS
- H. RÖMPP, J. FALBE: "Römpp Chemie-Lexikon, 9e édition", 1990, GEORG THIEME VERLAG, STUTTGART
- GESSNER G. HAWLEY:"The Condensed Chemical Dictionary, 9e édition", 1977, VAN NOSTRAND REINHOLD COMPANY, NEW YORK

## Description

La présente invention concerne un mélange d'huiles destiné à être utilisé dans des compositions cosmétiques, notamment un mélange d'huiles ayant une action de préparation de la peau à l'exposition solaire.

Le vieillissement prématuré de l'épiderme est dû en partie aux agressions du rayonnement UV générateur de radicaux libres.

On sait, par exemple de J-A-60193911, que certaines huiles végétales peuvent entrer dans la composition de produits cosmétiques résistants à l'eau à usage anti-solaire contenant en outre des filtres solaires dérivés de l'acide p-amino benzoïque.

La présente invention a pour but de proposer une composition lipidique pour produits cosmétiques ayant une action anti-vieillissement de la peau, c'est à dire anti-radicalaire, calmante et hydratante, tout en offrant une protection naturelle contre les radiations UV et étant naturellement stabilisée contre l'oxydation, c'est à dire ne contenant pas d'antioxydants ajoutés. Ladite composition lipidique se présente sous forme d'un mélange d'huiles.

Le mélange d'huiles selon l'invention est caractérisé par le fait qu'il contient au moins 40 % en poids d'huile d'amande d'abricot, 10 à 20 % en poids d'une huile contenant l'acide palmitoléique, 20 à 30 % en poids d'une huile choisie parmi l'huile de son de riz et l'huile de sésame et 15 à 25 % en poids d'huile d'olive.

Le mélange d'huiles contient de l'huile d'amande d'abricot. Nous avons constaté de manière inattendue que l'huile d'amande d'abricot, en sus de ses qualités cosmétiques, notamment sa douceur avait une grande stabilité propre contre l'oxydation.

En tant que huile contenant de l'acide palmitoléique, acide que l'on trouve dans le sébum, en particulier chez les sujets jeunes, on peut citer l'huile d'avocat ou l'huile de macadamia. L'huile d'avocat est préférée pour sa bonne pénétration et par le fait qu'elle contient des composés susceptibles d'agir comme filtre naturel des rayons UV.

La composition peut contenir de l'huile de son de riz qui est particulièrement riche en gamma-oryzanol ayant une activité antioxydante.
La composition peut contenir, en remplacement partiel ou total de l'huile de son de riz, de l'huile de sésame dont certains constituants inhibent spécifiquement la delta 5 désaturase, l'enzyme responsable de la biotransformation de l'acide dihomogamma-linolénique (DHGLA) en acide arachidonique (AA). Elle est donc susceptible d'avoir une action anti-inflammatoire dans la mesure où la formation des produits dérivés de l'AA comme, par exemple, le leucotriène B4, qui est pro-inflammatoire devrait diminuer au profit des produits dérivés du DHGLA, par exemple les prostaglandines de série 1 ayant une activité anti-inflammatoire.

La composition contient une quantité appréciable d'acide oléique qui confère au mélange lipidique une bonne stabilité à l'oxydation et à la photo-oxydation, ce qui évite la formation de radicaux oxygénés actifs.

La composition contient des huiles apportant les acides gras essentiels de la famille n-6 et pauvres en acides gras n-3, pour tenir compte de la plus grande sensibilité à l'oxydation de ceux de la famille n-3.

Ainsi, la composition contient une quantité appréciable d'acide linoléique. Cet acide est un constituant des céramides qui jouent le rôle important de barrière contre la deshydratation de l'épiderme. L'acide linoléique se révèle également actif contre l'hyperprolifération cellulaire associée à la carence en acides gras essentiels.

La composition moyenne des acides gras des triglycérides de la composition finale est la suivante:

| **Acides gras** | **% en poids** | | **% en poids** |
|---|---|---|---|
| C16:0 | 10-15 | de préférence | <13,3 |
| C16:1, n-7 | 1-2 | " | <1,5 |
| C18:0 | 1-2 | " | <1,5 |
| C18:1, n-9 | 30-65 | " | <63 |
| C18:2, n-6 | 20-30 | " | <28 |
| C18:3, n-3 (alpha) | 0,3-1 | " | <0,5 |
| C20:0 | <1 | " | <0,3 |
| C20:1 | <2 | " | <1,5 |

Sur la base de leur compositions respectives en acides gras et en constituants antioxydants naturels, les mélanges des huiles ci-après sont préférés:

| **Huile** | **% en poids** | | **% en poids** |
|---|---|---|---|
| Huile d'amande | au moins 40 | de préférence | 40 |
| d'abricot | | | |
| Huile d'avocat | 10-20 | " | 20 |
| Huile de son de riz | 20-30 | " | 20 |
| Huile d'olive | 15-25 | " | 20 |

L'invention concerne également un procédé de préparation d'un mélange d'huiles précédent, dans lequel on met en oeuvre des huiles partiellement raffinées, par dégommage, neutralisation, décoloration et wintérisation, caractérisé par le fait que l'on désodorise le mélange d'hniles dans des conditions ménagées, à environ 180 °C avec environ 1 % de vapeur vive par h et sous un vide d'environ 1-2 mbar pendant environ 3 h.

On peut ainsi maintenir une teneur appréciable en insaponifiables et en particulier ménager les tocophérols.

Le mélange d'huiles selon l'invention peut être avantageusement utilisé dans diverses compositions aqueuses ou anhydres pour le traitement de la peau, notamment dans des compositions aqueuses comme les fluides, crèmes et laits pour le visage, les mains et le corps, les crèmes et laits anti-solaires.

La composition cosmétique en question peut se présenter notamment sous la forme de solution, d'émulsion eau-dans-l'huile ou huile-dans-l'eau, de suspension ou d'aérosol.

Comme compositions cosmétiques anhydres incorporant la composition lipidique selon l'invention, on peut citer les huiles pour le corps, les baumes anhydres, les huilés anti-solaires et les rouges à lèvres.

Dans une telle composition cosmétique, la composition lipidique selon l'invention peut représenter 1 à 80%, et de préférence 5 à 60 % en poids.

Une telle composition cosmétique renferme généralement, en quantités appropriées, des adjuvants tels que, par exemple les émulsifiants, les agents anti-transpirants, les stabilisants, les conservateurs, les antioxydants, the filtres UV, les parfums, les colorants ou les émollients, les cires, les agents nacrants, les charges minérales ou organiques.

Les exemples ci-après illustrent l'invention. Dans ceux-ci, les pourcentages et parties sont pondéraux sauf indication contraire.

### Exemple 1

### Préparation du mélange d'huiles

On mélange sous agitation et sous azote les huiles partiellement raffinées ci-après dans les proportions indiquées.

| **Huile** | **% en poids** | | **% en poids** |
|---|---|---|---|
| Huile d'amande | au moins 40 | de préférence | 40 |
| d'abricot | | | |
| Huile d'avocat | 10-20 | " | 20 |
| Huile de son de riz | 20-30 | " | 20 |
| Huile d'olive | 15-25 | " | 20 |

Pour ce faire, dans un réacteur en acier inoxydable muni d'un système de double manteau à circulation de fluides pour thermostatisation et d'un agitateur à vitesse variable, on mélange les huiles dans les proportions indiquées ci-dessus en évitant des températures supérieures à 30°C.

On chauffe ensuite le mélange à 65 °C et on le traite avec 0,3 % d'acide citrique à 50 %, puis on ajoute 2 à 3 % d'eau et on sépare par centrifugation les gommes précipitées.

On met ensuite le mélange dégommé en contact avec 1 % de silicagel amorphe hydraté (TriSyl (R)) et 0,5 % silicagel amorphe hydraté (TriSyl 300(R)) à 80-85 °C pendant 20 min sous un vide de 50-80 mbar, pour le décolorer.

On désodorise enfin le mélange à 180°C pendant 3 h par entrainement à la vapeur avec 1 % de vapeur vive par h.

Le mélange présente un temps d'induction de 22,7 h au test Rancimat à 100° C.

### Exemple 2

| **Baume anhydre** | |
|---|---|
| ingrédient | % |
| Lanoline | 35 |
| Lanoline hydrogénée | 30 |
| Ozokérite | 3 |
| Composition lipidique selon l'exemple 1 | 20 |
| Cyclopentadiméthylsiloxane | 12 |

On obtient le produit anhydre précédent par mélange des constituants à 70°C, puis refroidissement sous brassage, jusqu'à la température ambiante.

### Exemple 3

| **Rouge à lèvres (anhydre)** | |
|---|---|
| Ingrédients | % |
| Esters d'alcools gras C8-C10 | 26 |
| Ozokérite | 10 |
| Cire de carnauba | 3 |
| Cire d'abeille | 3 |
| Pigment | 9 |
| Parfum | 0,1 |
| Huile de ricin | qsp 100 |
| Composition lipidique de l'exemple 1 | 6 |

On tamise les pigments. On mélange ensuite les constituants à 70°C, sauf le parfum. On laisse refroidir sous brassage jusqu'à 35 °C, puis on ajoute le parfum. On passe enfin la préparation au laminoir à trois cylindres.

### Exemple 4

| **Fond de teint** | |
|---|---|
| Ingrédients | % |
| Composition lipidique de l'exemple 1 | 4 |
| Mélange de mono-di-stéarate de glycéryle, acide stéarique, glycérine (40/50/5/5) | 3,3 |
| Mélange d'alcool de lanoline, huile de vaseline(15/85) | 3 |
| Mono-di-iso-stéarate de glycéryle | 1,8 |
| Palmitate d'isopropyle | 5 |
| Palmitate d'éthyl-2-hexyle | 5 |
| Oxyde de titane | 8,31 |
| Oxyde de fer brun | 0,73 |
| Oxyde de fer jaune | 1,7 |
| Oxyde de fer noir | 0,26 |
| p-Hydroxybenzoate de propyle | 0,1 |
| p-Hydroxybenzoate de méthyle | 0,1 |
| Parfum | 0,3 |
| Triéthanolamine | 1,2 |
| Silicate de magnésium et d'aluminium hydrate | 1,5 |
| Carboxyméthylcellulose de sodium | 0,14 |
| Cyclopentadiméthylsiloxane | 8 |
| Glycérine | 3 |
| Eau déminéralisée stérilisée | qsp 100 |
| Propylène glycol | 3 |
| Acide stéarique | 2,4 |

On mélange les pigments et on les tamise, puis on les incorpore dans la phase huileuse, préalablement chauffée à 70 °C. A part, on disperse le carboxyméthylcellulose de sodium dans l'eau. Lorsque la solution est homogène, on ajoute les autres composants de la phase aqueuse et on chauffe celle-ci à 75 °C. On émulsifie ensuite les deux phases avec homogénéisation rapide. On laisse refroidir sous brassage, on ajoute le parfum et la triéthanolamine à 35 °C, puis on homogénéise. On passe enfin la préparation au laminoir à trois cylindres.

### Exemple 5

| **Lait corporel protecteur hydratant** | |
|---|---|
| Ingrédients | % |
| Polysorbate 60 | 0,8 |
| Parfum | 0,3 |
| Glycérol stéarate et PEG 100 stéarate | 1 |
| Polyisobutène hydrogéné | 2 |
| Composition lipidique de l'exemple 1 | 8 |
| Acide stéarique | 1 |
| Glycérine | 3 |
| Carbopol 941 | 0,3 |
| Triéthanolamine | 0,3 |
| Eau + conservateur | qsp 100 |

On disperse le Carbopol 941 dans l'eau. Lorsque la solution est homogène, on ajoute les autres composants de la phase aqueuse et on chauffe celle-ci à 75 °C. A part, on mélange les constituants de la phase huileuse à 70 °C. On procède ensuite à l'émulsification des deux phases sous homogénéisation rapide. On laisse refroidir sous brassage et on ajoute le parfum, la triéthanolamine et le conservateur à 35 °C, puis on homogénéise. On laisse refroidir à la température ambiante et on conditionne.

### Exemple 6

| **Fluide de soin protecteur** | |
|---|---|
| Ingrédients | % |
| Méthyl glucose sesquistéarate | 2 |
| Composition lipidique de l'exemple 1 | 2 |
| Cyclométhicone | 13 |
| Parfum | 0,2 |
| PEG 20 méthyl glucose sesquistéarate | 2 |
| Gomme de xanthane | 0,2 |
| Polyacrylamide acide et C13-C14-isoparaffine et laureth 7 | 0,8 |
| | |
| Eau + conservateurs | qsp 100 |

On disperse la gomme de xanthane dans l'eau à 75 °C. A part, on mélange les constituants de la phase huileuse à 70 °C. On émulsifie alors les deux phases sous homogénéisation rapide. On laisse ensuite refroidir sous brassage, on ajoute le parfum et le conservateur à 35 °C, puis on homogénéise. On laisse enfin refroidir jusqu'à la température ambiante et on conditionne.

### Exemple 7

| **Crème de soin protectrice, émulsion huile-dans-l'eau** | |
|---|---|
| Ingrédients | % |
| PEG 20 stéarate | 1 |
| Glycéryl stéarate et PEG 100 stéarate | 1 |
| Acide stéarique | 1 |
| Alcool stéarylique | 2 |
| Composition lipidique de l'exemple 1 | 20 |
| Hydrolysat de protéine de soja | 0,2 |
| Glycérine | 3 |
| Carbopol 941 | 0,4 |
| Triéthanolamine | 0,4 |
| Eau + conservateur | qsp 100 |

On disperse le Carbopol 941 dans l'eau. Lorsque la solution est homogène, on ajoute les autres composants de la phase aqueuse et on chauffe celle-ci à 70 °C. A part, on mélange les constituants de la phase huileuse à 75 °C. On procède ensuite à l'émulsification des deux phases sous homogénéisation rapide. On laisse refroidir sous brassage et on ajoute le parfum à 35 °C, puis on homogénéise. On laisse refroidir à la température ambiante et on conditionne.

### Exemple 8

| **Crème de soins, émulsion eau-dans-l'huile** | |
|---|---|
| Ingrédients | % |
| Monoisostéarate de sorbitane | 5 |
| Cire microcristalline | 1 |
| Composition lipidique de l'exemple 1 | 19 |
| Esters d'acides gras en C8-C10 et d'alcools gras en C12-C18 | 1 |
| Gel de montmorillonite modifié et d'huile neutre (triglycérides d'acides caprylique et caprique) | 5 |
| Propylène glycol | 3 |
| Eau + conservateur | qsp 100 |

On mélange les constituants de la phase huileuse à 75 °C. A part, on chauffe les constituants de la phase aqueuse à 70 °C. Après émulsification des deux phases sous homogénéisation rapide, on laisse refroidir sous brassage jusqu'à la température ambiante et on conditionne.

### Exemple 9

| **Crème solaire, émulsion eau-dans -l'huile** | |
|---|---|
| Ingrédients | % |
| 4-(1,1-diméthyléthyl)-4'-méthoxydibenzoylméthane (PARSOL 1789) | 1,5 |
| p-méthylbenzylidène camphre (EUSOLEX 6300) | 4,5 |
| | |
| Mélange d'alcool cétylstéarylique et d'alcool cétylstéarylique oxyéthyléné à 33 moles d'oxyde d'éthylène | 7,3 |
| Mélange de mono- et distéarate de glycérol | 2,1 |
| Mélange d'huiles de l'exemple 1 | 31,4 |
| Polydiméthyl siloxane | 1,6 |
| Alcool cétylique | 1,6 |
| Eau | qsp 100 |

On prépare cette émulsion selon les techniques classiques en dissolvant les filtres dans la phase grasse contenant les agents émulsionnants, en chauffant cette phase grasse vers 80-85° C et en ajoutant, sous vive agitation, l'eau préalablement chauffée vers 80° C.

## Revendications

1. Mélange d'huiles pour produits cosmétiques ayant une action anti-vieillissement de la peau, c'est à dire anti-radicalaire, calmante et hydratante, tout en offrant une protection naturelle contre les radiations UV et étant naturellement stabilisé contre l'oxydation, c'est à dire ne contenant pas d'antioxydants ajoutés, **caractérisée par le fait qu'**il contient au moins 40 % en poids d'huile d'amande d'abricot, 10 à 20 % en poids d'une huile contenant l'acide palmitoléique, 20 à 30 % en poids d'huile de son de riz, d'huile de sésame ou d'un mélange de ces huiles et 15 à 25% en poids d'huile d'olive.

2. Mélange d'huiles selon la revendication 1, **caractérisé par le fait que** la composition moyenne des acides gras des triglycérides est la suivante:
| Acides gras | % en poids |
|---|---|
| C16:0 | 10-15 |
| C16:1, n-7 | 1-2 |
| C18:0 | 1-2 |
| C18:1, n-9 | 50-65 |
| C18:2, n-6 | 20-30 |
| C18:3, n-3 (alpha) | 0,3-1 |
| C20:0 | <1 |
| C20:1 | <2 |

3. Procédé de préparation d'un mélange d'huiles selon la revendication 1, dans lequel on met en oeuvre des huiles partiellement raffinées par dégommage, neutralisation, décoloration et wintérisation, **caractérisé par le fait que** l'on désodorise le mélange d'huiles dans des conditions ménagées, à 180° C pendant 3 h par entrainement à la vapeur avec 1 % de vapeur vive par h sous un vide de 1-2 mbar.

4. Utilisation d'un mélange d'huiles selon la revendication 1 dans la fabrication d'une composition cosmétique.

5. Utilisation selon la revendication 4, dans laquelle la composition cosmétique se présente sous forme aqueuse, de solution, de suspension ou d'aérosol, d'émulsion eau-dans-l'huile, notamment de crème ou huile-dans-l'eau, notamment de lait ou sous forme anhydre, notamment de baume, d'huile pour le corps, d'huile anti-solaire ou de rouge à lèvres et contient également au moins un adjuvant cosmétique choisi dans le groupe formé par les émulsifiants, les agents anti-transpirants, les stabilisants, les conservateurs, les antioxydants, les filtres UV, les parfums, les colorants, les émollients, les agents nacrants, les cires et les charges organiques ou minérales.

## Claims

1. Mixture of oils for cosmetic products that has anti-ageing action on the skin, that is to say free-radical-scavenging, soothing and moisturizing action, while at the same time offering natural protection against UV radiation and being naturally stabilized against oxidation, that is to say containing no added antioxidants, **characterized in that** it contains at least 40% by weight of apricot kernel oil, 10% to 20% by weight of an oil containing palmitoleic acid, 20% to 30% by weight of rice bran oil, sesame oil or a mixture of these oils, and 15% to 25% by weight of olive oil.

2. Mixture of oils according to Claim 1, **characterized in that** the mean fatty acid composition of the triglycerides is as follows:
| **Fatty acids** | **% by weight** |
|---|---|
| C16:0 | 10-15 |
| C16:1, n-7 | 1-2 |
| C18:0 | 1-2 |
| C18:1, n-9 | 50-65 |
| C18:2, n-6 | 20-30 |
| C18:3, n-3 (alpha) | 0.3-1 |
| C20:0 | < 1 |
| C20:1 | < 2 |

3. Process for preparing a mixture of oils according to Claim 1, in which oils that are partially refined, by degumming, neutralization, decolorization and winterization, are used, **characterized in that** the mixture of oils is deodorized under controlled conditions, at 180°C for 3 hours by vapour entrainment with 1% live steam per hour under a vacuum of 1-2 mbar.

4. Use of a mixture of oils according to Claim 1, in the manufacture of a cosmetic composition.

5. Use according to Claim 4, in which the cosmetic composition is in aqueous form, in the form of a solution, a suspension or an aerosol, a water-in-oil emulsion, especially a cream, or an oil-in-water emulsion, especially a milk, or in anhydrous form, especially in the form of a balm, a body oil, an antisun oil or a lipstick, and also contains at least one cosmetic adjuvant chosen from the group formed by emulsifiers, antiperspirants, stabilizers, preserving agents, antioxidants, UV screening agents, fragrances, dyes, emollients, pearlescent agents, waxes and organic or mineral fillers.

## Patentansprüche

1. Ölmischung für kosmetische Produkte mit einer Wirkung gegen das Altern der Haut, d.h. gegen freie Radikale, und mit beruhigender und hydratisierender Wirkung, die gleichzeitig einen natürlichen Schutz gegen UV-Strahlen bietet und natürlich gegenüber Oxidation stabilisiert ist, d.h. keine zugefügten Antioxidantien enthält, **dadurch gekennzeichnet, dass** sie mindestens 40 Gew.-% Aprikosenkernöl, 10 bis 20 Gew.-% Palmitoleinsäure enthaltendes Öl, 20 bis 30 Gew.-% Reiskleieöl, Sesamöl oder einer Mischung dieser Öle und 15 bis 25 Gew.-% Olivenöl enthält.

2. Ölmischung nach Anspruch 1, **dadurch gekennzeichnet, dass** die mittlere Zusammensetzung der Fettsäuren der Triglyceride die folgende ist:
| Fettsäuren | Gew.-% |
|---|---|
| C₁₆:0 | 10-15 |
| C₁₆:1,n-7 | 1-2 |
| C₁₈:0 | 1-2 |
| C₁₈:1,n-9 | 50-65 |
| C₁₈:2,n-6 | 20-30 |
| C₁₈:3,n-3(alpha) | 0,3-1 |
| C₂₀:0 | < 1 |
| C₂₀:1 | < 2 |

3. Verfahren zur Herstellung einer Ölmischung nach Anspruch 1, bei dem man Öle einsetzt, die durch Entfernung der Gummen, Neutralisierung, Entfärbung und Winterisierung partiell raffiniert wurden, **dadurch gekennzeichnet, dass** man die Ölmischung 3h unter schonenden Bedingungen bei 180°C durch Mitnahme durch Dampf mit 1% Frischdampf pro h unter einem Unterdruck von 1-2 mbar desodoriert.

4. Verwendung einer Ölmischung nach Anspruch 1 bei der Herstellung einer kosmetischen Zusammensetzung.

5. Verwendung nach Anspruch 4, bei der die kosmetische Zusammensetzung in wässriger Form, in Form von Lösung, Suspension oder Aerosol, Wasser-in-Öl-Emulsion, insbesondere Creme, oder Öl-in-Wasser-Emulsion, insbesondere Milch, oder in wasserfreier Form vorliegt, und zwar insbesondere in Form von Balsam, Körperöl, Sonnenschutzöl oder Lippenstift, und ferner mindestens einen kosmetischen Zusatz enthält, der aus der Gruppe ausgewählt ist, die aus folgenden Stoffen besteht: Emulgatoren, Schweißschutzmittel, Stabilisatoren, Konservierungsmittel, Antioxidantien, UV-Filter, Parfums, Farbstoffe, Weichmacher, Perlglanzmittel, Wachse und organische oder mineralische Füllstoffe.
